# FASCICULE DE BREVET EUROPEEN

(11) **EP 3 765 373 B1**
(45) Date de publication et mention de la délivrance du brevet: **21.02.2024**
(21) Numéro de dépôt: 19716962.6
(22) Date de dépôt: 05.03.2019
(51) Int. Cl.: H04N 23/50, H04N 23/51, B65B 3/00, A61M 5/178, B65B 57/10, B65B 57/18, B65B 59/00, H04N 7/18

(54) **DISPOSITIF DE PRISE DE VUE ET SYSTÈME POUR L'ÉLABORATION SÉCURISÉE DE PRÉPARATIONS MÉDICAMENTEUSES**
BILDAUFNAHMEVORRICHTUNG UND SYSTEM ZUR SICHEREN HERSTELLUNG VON MEDIZINISCHEN PRÄPARATEN
IMAGE CAPTURE DEVICE AND SYSTEM FOR THE SECURE PRODUCTION OF MEDICINAL PREPARATIONS

(30) Priorité: 13.03.2018 FR 1852146
(43) Date de publication de la demande: 20.01.2021
(73) Titulaire: Eurekam, 17140 Lagord (FR)
(72) Inventeur: LE BACCON, Gaël, 17000 LA ROCHELLE (FR); CONAN, Olivier, 17440 AYTRE (FR)
(74) Mandataire: IP Trust
(86) Numéro de dépôt international: PCT/FR2019/050489
(87) Numéro de publication internationale: WO 2019/175491

(56) Documents cités:
- EP-A1- 2 830 304
- WO-A1-2013/019795
- WO-A1-2014/202859
- FR-A1- 3 007 611
- FR-A1- 3 007 612

## Description

### Domaine technique

L'invention se rapporte à un procédé d'élaboration sécurisée, à savoir contrôlée et assistée, de préparations médicamenteuses ainsi qu'à un système d'imagerie apte à mettre en oeuvre un tel procédé.

Le domaine d'application de l'invention concerne la fabrication sécurisée de préparations médicamenteuses permettant un contrôle libératoire de ces préparations. Ce contrôle libératoire est nécessaire, car les préparations médicamenteuses se déroulent selon des protocoles complexes, à partir de mélanges réactionnels entre composants, chaque composant ayant une concentration adaptée à un traitement personnalisé pour un patient donné.

Toute erreur sur la nature d'un composant ou sur sa quantité peut entraîner des conséquences graves sur le patient auquel la préparation est administrée, en particulier lorsqu'une substance active toxique intervient, telles que les préparations de cytotoxiques qui peuvent être utilisées dans les traitements anticancéreux.

Dans la pratique, les opérateurs peuvent travailler plusieurs heures de suite, par exemple dans les unités de préparation des hôpitaux, ce qui augmente sensiblement le risque d'erreur sur la composition ou sur les quantités des composants. La sécurisation des préparations résulte habituellement d'un double contrôle visuel : vérification des étapes clés de chaque préparation et report écrit sur un support de traçabilité approprié, généralement appelé « fiche de fabrication ».

Afin de garantir la qualité des préparations médicamenteuses, l'AFSSAPS (Agence Française de Sécurité Sanitaire des Produits de Santé) a édité les « Bonnes Pratiques de Préparation » qui constituent un texte de référence destiné aux pharmaciens. Les « Bonnes Pratiques de Préparation » précisent les trois obligations auxquelles doit obéir la mise en oeuvre des matières premières utilisées dans les préparations médicamenteuses :
- la méthode de mesure des quantités de matières premières est choisie en fonction de leur nature et de la quantité à mesurer ;
- la mesure du volume ou la pesée des quantités de matières premières fait l'objet d'enregistrements ;
- les matières premières sont identifiables de manière permanente au cours des opérations précitées.

Ainsi, lors de la préparation, la nature de chaque matière première utilisée ainsi que sa masse ou son volume sont vérifiés indépendamment soit par un moyen d'enregistrement automatique, soit par une seconde personne qualifiée au sens du Code de la Santé Publique, et la vérification est notée dans le dossier de lot de la préparation. Pour répondre aux « Bonnes Pratiques de Préparation » et pour la sécurité des patients, il est donc recommandé d'effectuer un double contrôle de la nature et des quantités des compositions utilisées dans chaque préparation.

### Etat de la technique antérieure

De la publication WO 2014/202859 A1 de la demanderesse, on connaît un procédé et dispositif de prise de vue pour l'élaboration sécurisée de préparations médicamenteuses et un support de positionnement d'objets associés.

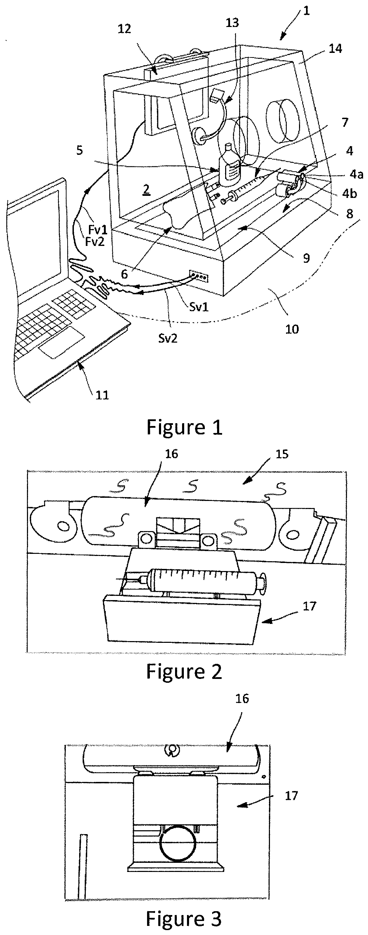

En référence à la vue en perspective de la figure 1, qui reprend la figure 1 de la publication précitée, il est maintenant rappelé le contexte de suivi de la préparation médicamenteuse développé par la demanderesse.

Une hotte portative 1 intègre un site 2 de préparation de médicaments. Ce site 2 est équipé d'un système d'imagerie 3 (non représenté) permettant d'élaborer de telles préparations de manière sécurisée. Ce système d'imagerie 3 comporte une caméra 4 dite de traitement. La focale de cette caméra 4 est réglée pour réaliser une mise au point sur des contenants et des outils d'administration de médicaments, ici un flacon 5, une poche de perfusion 6 contenant une solution saline, et une seringue 7. Dans l'exemple, le flacon 5 contient du cisplatine à diluer dans la poche de perfusion 6. Pour ce faire, un prélèvement est effectué par l'opérateur dans le flacon 5 de cisplatine à l'aide de la seringue 7 puis injecté dans la poche de perfusion 6. L'opération est reproduite jusqu'à l'obtention de la dilution voulue du principe actif dans la poche pour préparer, dans l'exemple, un composant de polychimiothérapie.

Avantageusement, un fond 8 de la hotte 1 possède une conformation de structure surfacique adaptée aux contours des objets, favorisant une dépose stabilisée et reproductible des flacons et seringues, et intègre un dispositif de rétroéclairage 9 permettant de mieux lire les gradations de la seringue 7.

La caméra de traitement 4 est positionnée au niveau du fond de hotte 8 légèrement au-dessus d'un plan de travail sur lequel est posée la hotte portative 1, c'est-à-dire sensiblement, dans l'exemple, au niveau ventral d'un l'opérateur (non représenté).

La caméra de traitement 4 est connectée à une unité de gestion numérique, un ordinateur portable 11 dans l'exemple. L'ordinateur 11 comporte principalement un processeur et des mémoires (non représentés) qui traitent des signaux vidéo Sv1 provenant de la caméra de traitement 4 pour fournir des images à un écran d'affichage 12 et les enregistrer.

L'écran d'affichage 12 permet alors de visualiser des informations correspondant à la préparation en cours à partir d'un flux vidéo Fv1 fourni par la caméra de traitement 4.

Une autre caméra 13 d'enregistrement et de visualisation, dite caméra de scène, possède une focale réglée pour une détection globale du site 2. L'objectif de la caméra de scène 13 est avantageusement positionné en un niveau supérieur 14 de la hotte 1 de sorte à permettre une vision globale du site 2 par transmission d'un flux vidéo Fv2 à l'écran d'affichage 12.

Les caméras de traitement 4 et de scène 13 fournissent des signaux vidéo S_{V1}, S_{V2} synchronisés par le processeur de l'ordinateur 11 afin de constituer une interface graphique dynamique pour un contrôle en temps réel et *a posteriori.*

La caméra de traitement 4 est avantageusement composée de deux caméras accolées 4a, 4 b, de focales réglées sur la détection d'objets - flacons et seringues en général - dans des gammes de dimensions complémentaires, inférieure à 3 cm et compris entre 3 et 307 cm dans l'exemple.

Les flux vidéo des caméras de traitement 4a et 4b sont analysés par un outil de reconnaissance de forme et de caractère, intégré au processeur de l'ordinateur 11.

L'analyse permet une identification automatique des objets utilisés, flacon 5 et seringue 7, par un traitement des premiers flux vidéo à l'aide de l'outil de reconnaissance. La détection des volumes de produit contenus dans les seringues et les flacons permet un contrôle non destructeur du principe actif mis en oeuvre dans la préparation.

Ainsi, toutes les données figurant sur du flacon 5, en particulier la concentration du principe actif, sont identifiées par ledit outil de reconnaissance. Ces données sont mémorisées dans l'ordinateur 11. Au cours de la préparation, des données d'analyse des contenus du flacon et de la seringue sont affichées sur l'écran 12 et enregistrées en vue de la validation de chaque étape de la préparation : quantité de liquide dans la seringue 7 et niveau de liquide dans le flacon 5.

Le processeur de l'ordinateur 11 recherche dans une mémoire, où est stocké un ensemble de prescriptions, la prescription correspondant à la préparation en cours. Pour ce faire, les prescriptions mémorisées sont indexées par étapes à l'aide d'un outil d'indexation numérique. Les étapes clés - qui sont les étapes spécifiques propres à chaque prescription - ont une indexation particulière.

Le processeur compare les données mémorisées de la préparation en cours fournies par les premières images enregistrées - nom des composants, quantités versées dans les seringues, etc. - et les étapes des prescriptions mémorisées. Dès qu'une étape clé de cette prescription est reconnue, la prescription correspondant à la préparation en cours est alors identifiée et affichée par ses étapes. Les informations sont sélectionnées en fonction de la prescription détectée parmi une liste de flacons disponibles et une liste de flacons utilisés mémorisées dans l'ordinateur en fonction de données fournies par le centre de gestion du laboratoire.

Un affichage d'alerte est intégré, ainsi que la possibilité d'interaction manuelle pour prélever ou injecter un volume déterminé, ou encore ajouter un flacon.

De plus, l'état d'avancement de la préparation est affiché en fonction des données du protocole mémorisé correspondant à la prescription identifiée : des informations concernant la gestion du volume contenu dans le flacon 5 et l'évolution des volumes du composant injectés/manquants/prescrits dans la seringue 7 sont affichées. Ces informations de gestion sont initiées par une incrémentation des volumes injectés et en conséquence du volume restant, du nombre d'injections, du nombre de flacons et des reliquats. Cette incrémentation est automatisée à partir de l'interface graphique des flux vidéo synchronisés des caméras 4 et 13. Une telle gestion permet de baisser sensiblement les erreurs dues aux manipulations.

L'affichage d'alerte est activé en temps réel dès que le flacon et/ou les volumes de composant détectés avant injection dans une étape ne sont pas conformes au protocole mémorisé de la prescription identifiée. Les étapes détectées en erreur sont identifiées par comparaison avec les étapes de la prescription. La non-conformité d'une étape clé déclenche une recherche d'erreur dans l'identification de la prescription qui a été sélectionnée.

L'affichage fournit alors des directives et l'alerte n'est supprimée et la préparation ne se poursuit que si la conformité entre les étapes de la préparation et celles constituant le protocole de ladite prescription est validée. L'avancement de la préparation est alors réactualisé en temps réel jusqu'à la validation finale. La préparation est ainsi sécurisée par une réactivité quasi instantanée et la reproductibilité des préparations est optimisée.

La validation des étapes détectées en erreur, en particulier des étapes clés de la préparation, permet de poursuivre la préparation jusqu'à son terme.

Ainsi, la préparation peut se faire sous hotte fixe ou tout environnement adapté pour installer le système d'imagerie.

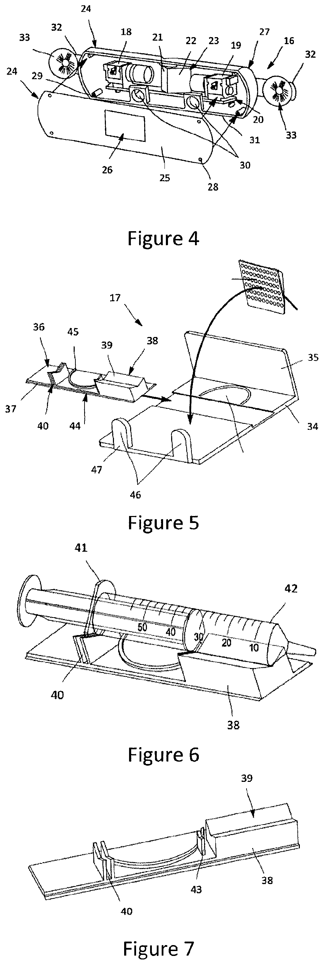

En référence aux figures 2 à 7, qui reprennent les figures 4 à 9 de la publication précitée, il est rappelé un exemple de système de prise de vue mettant en oeuvre le procédé d'élaboration sécurisée de préparations médicamenteuses est décrit.

Ce système est disposé de part et d'autre d'une baie transparente 15 d'une enceinte d'élaboration sécurisée d'une préparation médicamenteuse, et comprend un dispositif de prise de vue 16 disposé à l'extérieur de l'enceinte contre la paroi transparente 15, et un support 17 de positionnement d'objets disposé à l'intérieur de l'enceinte en vis-à-vis du dispositif de prise de vue.

Plus précisément, comme mieux visible sur la figure 4, le dispositif de prise de vue 16 comprend deux caméras de traitement 18, 19 disposées aux deux extrémités d'une embase 20 allongée et rectangulaire, un élément réfléchissant 21, 22 pour chacun des appareils de prise de vue 18, 19, disposés à mi-longueur de l'embase 20.

Les deux éléments réfléchissants 21, 22 sont orientés le long de l'axe défini par les deux caméras, de façon à réfléchir en direction de la caméra correspondante, des images du support 17 de positionnement d'objets.

Plus précisément, ces deux éléments réfléchissants sont formés par les deux faces rectangulaires adjacentes d'un prisme droit à base triangulaire 23, le prisme étant monté sur l'embase par l'une de ses bases triangulaires.

Les deux caméras 18, 19 présentent des objectifs de focales différentes permettant de faire le point sur des objets de tailles différentes se trouvant sur le support de seringues et flacons. Par exemple, la caméra de droite peut être consacrée à la mise au point pour des flacons et seringues de petite taille avec un objectif de 12 mm, celle de gauche pouvant être consacrée à la mise au point pour des flacons et seringues de grande taille avec un objectif de 8 mm.

Afin de doter le dispositif de prise de vue de faibles dimensions, les deux caméras présenteront elles-mêmes de faibles dimensions, par exemple 47x 29 x 29 mm (L x l x h). Un exemple de caméras convenant à cette utilisation est de la marque Imaging source^{®}, référence DFK23F445.

L'embase allongée 20 est fixée à l'intérieur d'un coffret 24 comprenant une face avant 25 plane et sensiblement rectangulaire, dont les deux côtés courts sont légèrement arrondis, et munie d'une fenêtre centrale de visibilité 26 sensiblement rectangulaire, et une coque arrière allongée 27 à l'intérieur de laquelle l'embase 20 est solidairement fixée, par son bord arrière longitudinal, le prisme 23 se retrouvant dans l'ouverture de la fenêtre 26 lorsque la face avant 25 du coffret referme la coque 27, afin que les images du support de seringues et flacons parviennent via ce prisme, aux deux caméras 18, 19.

La face avant 25 peut être fixée à la coque 27 au moyen de vis traversant des trous 28 prévus à cet effet aux quatre coins de la face avant 25 et venant se visser dans un fourreau correspondant 29 prévu à cet effet sur la paroi interne de la coque arrière 27.

La coque arrière 27 comprend en outre deux pattes 30 s'étendant verticalement depuis son bord latéral inférieur 31, ces pattes étant pourvues d'un évidemment central circulaire d'accueil d'un moyen magnétique de forme complémentaire (pastille métallique ou aimantée, non représentée), destiné à retenir contre la baie vitrée 15 et la face avant 25 du coffret, le support pour seringues et flacons 17 qui est pourvu de moyens aimantés complémentaires.

La coque 27 comprend en outre deux lames latérales rigides 32 fixées sur la face externe de sa paroi arrière et dépassant de part et d'autre de ses extrémités, ces deux lames 32 portant deux ventouses 33 dirigées vers l'avant afin d'être fixées sur la baie vitrée de l'enceinte.

D'autre part, conformément à la figure 5, le support 17 pour seringues et flacons comprend une base 34 plane de support, un écran incliné 35 dressé en arrière de la base destiné à former un fond blanc, et un porte-seringue 36 monté à translation vis-à-vis de la base 34 entre une position désengagée indépendante de la base 34 (illustrée sur la figure 5) et une position engagée solidaire de la base 34 (illustrée sur la figure 2).

Plus précisément, le porte seringue 36 comprend une plaque rectangulaire 37, à une extrémité de laquelle fait saillie un pavé 38 de forme sensiblement rectangulaire, mais dont la paroi supérieure est en creux longitudinal 39 (la paroi supérieure est composée de pans inclinés convergeant l'un vers l'autre et se rejoignant par un bord commun, définissant une section transversale en forme de « M ») afin de servir de support pour le corps d'une seringue, et de retenir latéralement par les pans inclinés, ce corps de seringue.

À l'autre extrémité, la plaque 37 du porte seringue 36 comprend un chevalet 40 de retenue des ailes 41 d'une seringue 42. Le chevalet 40 est formé par deux parois parallèles, séparées l'une de l'autre d'une distance suffisante pour accueillir les ailes 41 de la seringue 42 (voir figure 6), et présentent, à l'image du pavé 38, un contour en forme de « M » afin de supporter et retenir latéralement la partie de seringue à proximité des ailes 41.

Conformément à la figure 7, il est prévu, pour le support de seringues de petites tailles, un chevalet supplémentaire 43 entre le pavé 38 et le chevalet principal 40, en étant plus proche du pavé 38, et se présentant sous la forme d'une unique paroi 43 à contour en forme de « M », dont le sillon se retrouve au même niveau que celui du pavé 38.

La plaque 37 du porte-seringue 36 comprend, conformément à la figure 5, deux bords latéraux amincis 44 susceptibles de coulisser dans des gorges formées sur les bords latéraux d'un creux d'accueil de la plaque ménagé dans la base 34, afin de pouvoir engager par coulissement le support de seringues dans la base jusqu'à une position engagée (figure 2).

Dans cette position engagée, le support de seringues 36 et la base 34 définissent ensemble un relief 45 d'accueil pour le cul d'un flacon.

En effet, les surfaces supérieures de la base 34 et de la plaque 37 du porte seringue 36 sont coplanaires lorsque le porte seringues occupe sa position engagée et comprennent des creux complémentaires 45 d'accueil d'un flacon en forme de demi disque, et qui forment lorsque le porte seringue occupe sa position engagée, un creux d'accueil circulaire du cul d'un flacon, ce creux se retrouvant disposé entre le chevalet de retenue 40 et le pavé d'appui 38. Bien entendu, un autre relief, tel qu'une nervure circulaire, peut remplir la même fonction.

La base 34 porte les moyens magnétiques complémentaires 46 de ceux prévus sur les pattes 30 du dispositif de prise de vue. Plus précisément, ces moyens magnétiques (pastille aimantée ou métallique, suivant celle utilisée pour les moyens magnétiques du dispositif de prise de vue) sont logés à l'intérieur de deux ergots 47 faisant saillie verticalement du bord de la base 34 opposé à l'écran 35, et présentant une paroi avant plane pouvant se plaquer contre la face interne de la baie vitrée, au niveau des pattes correspondantes 30 du dispositif de prise de vue.

Le système tel que décrit ci-dessus permet la mise en oeuvre du procédé d'élaboration sécurisé de préparations médicamenteuse, même lorsque des seringues et des flacons de différentes tailles sont utilisés, grâce à la pluralité d'appareils de prise de vue à objectifs différents utilisés et aux éléments réfléchissants permettant à ces appareils de recueillir des images de la même zone : le support de seringues et de flacons.

Cet objectif est en outre atteint avec un encombrement faible du fait de l'utilisation d'un prisme central pour les deux appareils de prise de vue.

Toutefois, la demanderesse a continué d'innover.

La demanderesse a noté que le positionnement du système décrit dans la publication précitée pouvait être imprécis, notamment du fait de l'utilisation de ventouse. En effet, à l'installation, une calibration est nécessaire. Le nettoyage de la baie 15 peut imposer le retrait du dispositif de prise de vue et donc de ses ventouses. Il est ensuite alors difficile de le repositionner à l'identique, ce qui peut entraîner des problèmes de prises de vue.

La demanderesse a en outre noté que son système n'était pas à différentes inclinaisons de baies transparentes par rapport au plan de travail. En effet, entre deux sites prédéterminés sur lesquels la préparation médicamenteuse doit être réalisée, les inclinaisons des baies peuvent être différentes, obligeant la demanderesse à adapter son système à chacune des inclinaisons de baies.

La demanderesse a aussi noté que les opérateurs sont sujets à des troubles musculosquelettiques (TMS) et que la présence de l'écran implique une gestuelle entraînant des tendinites de la coiffe des rotateurs de l'épaule de l'opérateur lorsque l'opérateur met en oeuvre le produit.

### Exposé de l'invention

Un but de l'invention est notamment de remédier à tout ou partie des inconvénients précités.

Selon un premier aspect de l'invention, il est proposé un dispositif de prise de vue selon la revendication 1.

Aussi, il devient alors possible de rendre plus précis le positionnement selon l'invention. On facilite alors le nettoyage de la baie 15 et le repositionnement à l'identique du système.

Les moyens de positionnement peuvent coopérer avec des moyens magnétiques complémentaires disposés sur la partie amovible.

Avantageusement, le dispositif selon l'invention peut en outre comporter des moyens de sélection de l'angle formé entre la base et la demi-embase.

Selon une possibilité, les deux zones couvertes par les deux appareils d'une même paire d'appareils sont spatialement décalées, et les appareils présentent des objectifs de focales différentes permettant de faire la mise au point sur des objets de tailles différentes se trouvant dans la zone d'élaboration de la préparation médicamenteuse.

Selon un mode de réalisation, la partie amovible comporte un capot qui est lui-même amovible par rapport à ladite partie amovible. Il devient alors possible de régler différents paramètres des caméras ainsi que leur position angulaire, sans avoir à démonter tout le système, ce qui n'était pas possible selon l'art antérieur.

A titre d'exemple, les deux éléments réfléchissants peuvent être formés par des surfaces planes.

Selon un deuxième aspect de l'invention, il est proposé un système d'imagerie sécurisé, comprenant un dispositif de prise de vue selon le premier aspect de l'invention, ou de l'un ou plusieurs de ses perfectionnements, un support de positionnement d'objets, installés sur une enceinte d'élaboration de préparations médicamenteuses, le support de positionnement d'objets étant disposé à l'intérieur de l'enceinte, et le dispositif de prise de vue à l'extérieur de celle-ci contre une baie transparente de l'enceinte, et à une position telle que des images du support de positionnement d'objets, parviennent aux appareils de prise de vue du dispositif.

Avantageusement, les moyens de fixation du support de positionnement d'objets peuvent être des moyens magnétiques qui coopèrent à travers l'enceinte de préparation médicamenteuse, avec les moyens de positionnement disposés sur la partie fixe du dispositif de prise de vue.

De préférence, les moyens de fixation du support de positionnement d'objets sont des moyens magnétiques qui coopèrent avec les moyens magnétiques complémentaires disposés sur la partie amovible du dispositif de prise de vue.

A titre d'exemple, le support de positionnement d'objet peut en outre comporter une base plane de support des objets et un écran dressé en arrière de la base plane, l'écran étant amovible par rapport à la base plane, l'écran comportant des moyens magnétiques complémentaires de moyens magnétiques logés dans la base plane.

### Présentation des figures

D'autres données, caractéristiques et avantages de la présente invention apparaîtront à la lecture de la description de mises en oeuvre et de modes de réalisation nullement limitatifs, au regard de dessins annexés sur lesquels :
- la figure 1 est une vue en perspective d'un site de préparations médicamenteuses équipé d'un exemple de système d'imagerie sécurisé de telles préparations ;
- la figure 2 est une vue en perspective de face d'un système de prise de vue de seringues et de flacons de tailles différentes, se positionnant sur la baie transparente d'une enceinte de préparation, un support pour seringues et flacons, interne à l'enceinte, et un dispositif de prise de vue externe à l'enceinte,
- la figure 3 est une vue en perspective de dessus du système de la figure 2,
- la figure 4 est une vue éclatée du dispositif de la figure 2,
- la figure 5 est une vue éclatée du support pour seringues et flacons de la figure 2, représentant un porte-seringue en position désengagée vis-à-vis d'une base du support,
- la figure 6 est une vue en perspective du côté droit du support soutenant une seringue,
- la figure 7 est une vue en perspective du côté gauche du support sans seringue,
- la figure 8 est une vue en perspective de l'avant, du dessus et depuis la droite d'un éclaté d'un système selon l'invention,
- la figure 9 est une vue en perspective de l'avant, du dessus et depuis la droite d'un éclaté d'une partie fixe d'un dispositif de prise de vue du système selon l'invention,
- la figure 10 est une vue en perspective de l'avant et de droite, d'un sous-ensemble mécanique d'une partie amovible du dispositif de prise de vue,
- la figure 11 est une représentation schématique éclatée du sous-ensemble mécanique aussi illustré sur la figure 10,
- la figure 12 est une vue en perspective de l'avant, du dessus et depuis la droite d'un éclaté de la partie amovible du dispositif de prise de vue.
- la figure 13 est une vue en perspective de l'arrière, du dessous, et de droite de l'éclaté aussi représenté sur la figure 12,
- la figure 14 est une vue en perspective de l'avant, du dessus et depuis la droite d'un support d'objets du système.
- la figure 15 est une vue en perspective du côté droit d'un éclaté du support d'objets aussi représenté sur la figure 14.

### Description des modes de réalisation

Les modes de réalisation décrits ci-après n'étant nullement limitatifs, on pourra notamment considérer des variantes de l'invention ne comprenant qu'une sélection de caractéristiques décrites, par la suite isolées des autres caractéristiques décrites, si cette sélection de caractéristiques est suffisante pour conférer un avantage technique ou pour différencier l'invention par rapport à l'état de la technique antérieure. Cette sélection comprend au moins une caractéristique, de préférence fonctionnelle sans détails structurels, ou avec seulement une partie des détails structurels si cette partie uniquement est suffisante pour conférer un avantage technique ou pour différencier l'invention par rapport à l'état de la technique antérieure.

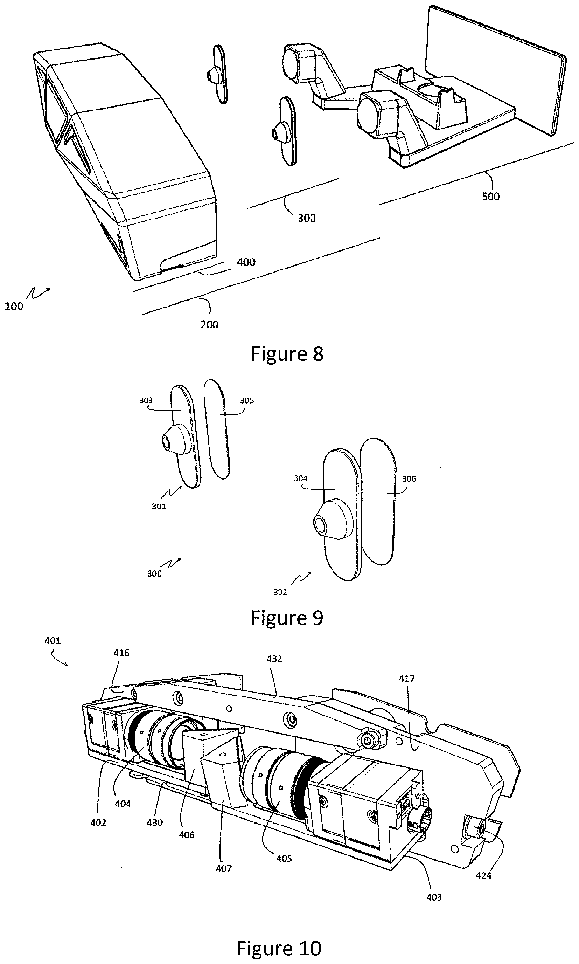

En référence aux figures 8 à 14, il est maintenant décrit un exemple de système de prise de vue 100 mettant en oeuvre le procédé d'élaboration sécurisée de préparations médicamenteuses précédemment décrit.

Comme précédemment exposé, ce système est adapté pour être disposé de part et d'autre d'une baie transparente 15 d'une enceinte d'élaboration sécurisée d'une préparation médicamenteuse.

Le système comprend un dispositif 200 de prise de vue disposé à l'extérieur de l'enceinte contre la paroi transparente 15, et un support 500 de positionnement d'objets disposé à l'intérieur de l'enceinte en vis-à-vis du dispositif 200 de prise de vue.

Plus précisément, le dispositif de prise de vue 200 comporte :
- une partie fixe 300 destinée à être fixée à demeure sur la baie transparente 15 de la zone d'élaboration de la préparation médicamenteuse,
- une partie amovible 400, destinée à être fixée de manière amovible, sur la partie fixe 300.

Le système de prise de vue 100 comporte un plan de symétrie principal, vertical, et selon la direction, dite longitudinale, comporte, successivement, de l'avant à l'arrière, le dispositif 200 et le dispositif 500.

En référence à la figure 9, la partie fixe 300 est maintenant décrite. Elle comporte deux moyens de positionnement 301 et 302 destinés à être fixés à demeure sur la baie transparente 15.

Par convention, le moyen de positionnement 301 est dit positionné à gauche et le moyen de positionnement 302 est dit positionné à droite.

Chacun des deux moyens de positionnement 301, respectivement 302, comporte un plot inox magnétique 303, respectivement 304.

Par plot inox magnétique, la présente description vise un plot en acier inoxydable magnétique, par exemple de type ferritique ou martensitique.

Les plots inox magnétiques sont destinés à être fixés à demeure sur la face extérieure de la baie 15.

A cet effet, chacun des deux ensembles 301, respectivement 302, comporte un adhésif double face 305, respectivement 306, de même forme oblongue que le plot inox magnétique 303, respectivement 304. Un côté de l'adhésif 305, respectivement 306 est collé sur le plot inox magnétique 303, respectivement 304. L'autre côté de l'adhésif 305, respectivement 306, est destiné à être collé sur la face extérieure de la paroi transparente 15.

Comme mieux visible sur les figures 10 à 11, la partie amovible 400 comporte un ensemble mécanique interne 401.

L'ensemble mécanique interne 401 comporte deux demi-embases 402 et 403, respectivement gauche et droite, allongées et rectangulaires.

Chacune des deux demi-embases, respectivement 402 et 403, est prévue pour recevoir une caméra de traitement, respectivement 404 et 405 et un élément réfléchissant, respectivement 406 et 407, pour chacun des appareils de prise de vue 404, 405.

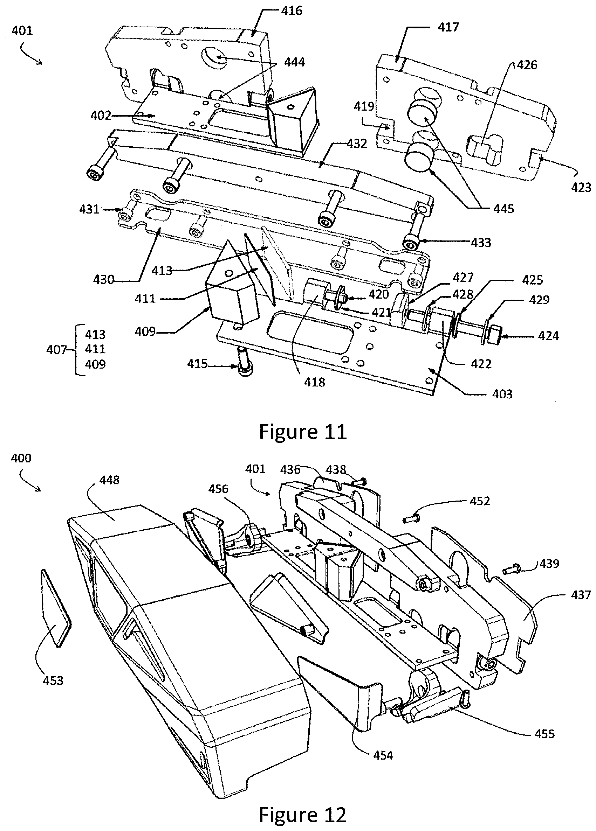

Dans une position telle que visible sur la figure 12, les deux demi-embases 402 et 403 sont disposées côte à côté le long d'une de leur largeur, la demi-embase 402 à gauche de la demi-embase 403 :
- la caméra de traitement 404 est disposée au niveau de l'extrémité gauche de la demi-embase 402 et est orientée vers la droite,
- la caméra de traitement 405 est disposée au niveau de l'extrémité droite de la demi-embase 403 et est orientée vers la gauche,
- les deux éléments réfléchissants 406 et 407 se situent respectivement à droite et à gauche de la demi-embase 402 et 403.

Les deux éléments réfléchissants, respectivement 406 et 407, sont orientés de façon à réfléchir en direction de la caméra correspondante, respectivement 404 et 405, des images du support 500 de positionnement d'objets.

De manière encore plus précise, en référence à la figure 11, l'élément réfléchissant 407 comporte un support principal 409 à face rectangulaire qui est collé à un adhésif double face 411, lui-même collé à un miroir 413.

L'élément réfléchissant 407 est fixé sur la demi-embase 403 par une vis 415, rendant possible un réglage angulaire du support principal 409, par rapport à un axe perpendiculaire à la demi-embase 403.

L'élément réfléchissant 406 est composé d'éléments semblables en symétrie, par rapport au plan de symétrie principal.

Les deux caméras 404, 405 présentent des objectifs de focales différentes permettant de faire le point sur des objets de tailles différentes se trouvant sur le support de seringues et flacons. Par exemple, la caméra de droite peut être consacrée à la mise au point pour des flacons et seringues de petite taille avec un objectif de 12 mm, celle de gauche pouvant être consacrée à la mise au point pour des flacons et seringues de grande taille avec un objectif de 8 mm.

Afin de doter le dispositif de prise de vue de faibles dimensions, les deux caméras présenteront elles-mêmes de faibles dimensions, par exemple 47x 29 x 29 mm (L x l x h). Un exemple de caméras convenant à cette utilisation est de la marque Imaging source^{®}, référence DFK23F445.

Chacune des demi-embases, respectivement 402 et 403, est montée à rotation, le long d'un axe perpendiculaire au plan de symétrie principal, et disposé sur une partie arrière et au-dessus de son embase, sur une base, respectivement une base gauche 416 et une base droite 417.

La liaison entre l'embase droite 403 et la base droite 417 est formée par deux liaisons pivots selon un même axe, les deux liaisons étant axialement maintenues en butée.

Un premier oeil 418 rapporté sur l'embase 403, du côté gauche de l'embase 403, et un premier logement pour axe 419 formé sur la base droite 417, du côté gauche de la base droite 417, coopèrent avec une goupille 420 pour former une première charnière entre l'embase 403 et la base droite 417. Une rondelle 421, de préférence conique et striée, est disposée radialement autour de la goupille 420, entre le premier oeil 418 et le logement pour axe 419. La base droite 417 comporte un logement pour le premier oeil 418.

Un deuxième oeil 422 rapporté sur l'embase 403, du côté droit de l'embase 403, et un deuxième logement pour axe 423 rapporté sur la base droite 417, du côté droit de la base droite 417, coopèrent avec une vis 424 pour former une deuxième charnière entre l'embase 403 et la base droite 417. Une rondelle 425, de préférence conique et striée, est disposée radialement autour de la vis 424, entre le deuxième oeil 422 et le logement pour axe 423. La base droite 417 comporte un logement 426 pour le deuxième oeil 422.

Le logement 426 est en outre conformé pour y loger un écrou 427 prisonnier recevant la vis 424. Une rondelle 428, de préférence de type nylon est prévue pour être montée radialement autour de la vis 424, entre l'écrou prisonnier et le deuxième oeil 422.

Une autre rondelle 429, de préférence de type nylon, est prévue pour être montée radialement autour de la vis 424, entre le logement pour axe 423 et la tête de la vis 424.

De la même manière, la liaison entre la demi-embase gauche 402 et la base gauche 416 est formée par deux liaisons pivots selon un même axe, les deux liaisons étant axialement maintenues en butée.

On comprend que la vis 424 et l'écrou 427 forment un moyen de réglage de la position angulaire de la demi-embase droite 403 par rapport à la base droite 417.

La position angulaire de la demi-embase gauche 402 par rapport à la base gauche 416 est également réglable, par exemple au moyen d'une vis et d'un écrou prisonnier.

L'ensemble mécanique interne 401 comporte en outre un bras de liaison basse 430, allongé et rectangulaire. Le bras de liaison bas 430 est solidairement fixé, par un bord arrière longitudinal, au bas de chacune des bases, respectivement base gauche 416 et base droite 417, du côté avant de la base, au moyen d'éléments de fixation, tels que des vis 431.

L'ensemble mécanique interne 401 comporte en outre un bras de liaison haute 432. Le bras de liaison haut 432 est solidement fixé, en haut de chacune des bases, respectivement base gauche 416 et base droite 417, du côté avant de la base, au moyen d'éléments de fixations, tels que des vis 433.

Le côté avant de la base est le côté sur lequel les caméras sont situées par rapport au plan de la base. Réciproquement, le côté arrière de la base est le côté sur lequel les caméras sont situées par rapport au plan de la base.

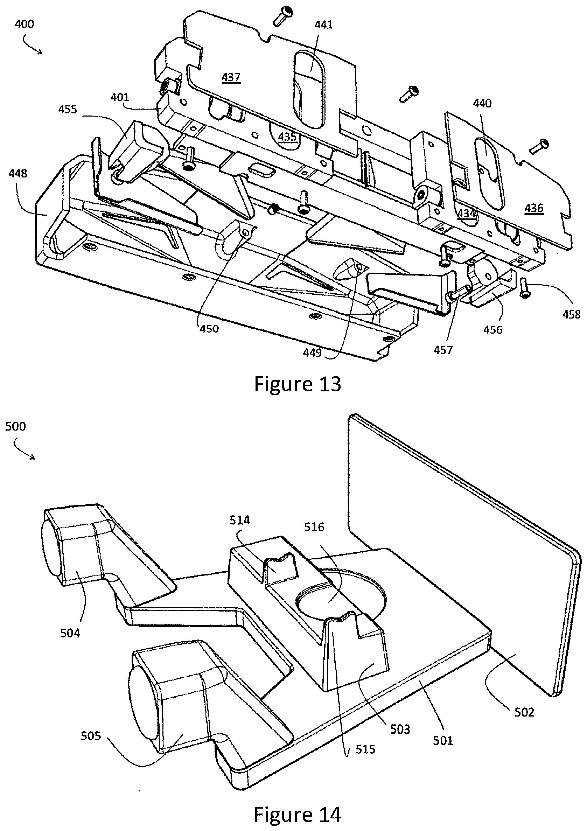

Comme mieux visible en Figure 13, chacune des bases 416, respectivement 417, comporte, du côté arrière de la base, un logement vertical 434, respectivement 435, de forme oblongue présentant un trou de centrage de forme tronconique en son centre, d'axe perpendiculaire à la forme oblongue.

Le dispositif mécanique de prise de vue 400 comporte en outre deux plaques de mousses, respectivement droite 436 et gauche 437, de même section que la section arrière de la base gauche 416 et la base droite 417.

Chacune des plaques de mousse, respectivement droite 436 et gauche 437, est fixée au côté arrière de sa base, respectivement base gauche 416 et base droite 417, au moyen d'un adhésif.

Chacune des plaques de mousse, respectivement droite 436 et gauche 437, comporte une fenêtre, respectivement 440 et 441, de forme coopérant avec la forme oblongue du logement vertical associé, respectivement 434 et 435.

Le dispositif mécanique de prise de vue 400 comporte en outre un capot 448 présentant une face frontale, des faces supérieures et inférieures, ainsi que des faces latérales, gauche et droite.

Le capot 448 est agencé pour loger l'ensemble mécanique interne 401. A cet effet, la face arrière de la face frontale présente, dans sa partie supérieure, trois logements 449, 450, 451 (non référencé sur les figures), avec insert, perpendiculaires à la face frontale du dispositif de prise de vue, et s'étendant vers l'arrière.

Des vis 438 et 439 (Figure 12) traversent d'arrière en avant l'ensemble mécanique 401 et sont vissées, respectivement, dans les logements avec insert 449 et 451.

Une vis 452 traverse d'arrière en avant l'ensemble mécanique 401 et est vissée dans le fourreau 450.

En référence à la figure 9, chacun des deux ensembles 301, respectivement 302, coopère mécaniquement avec le logement vertical 434, respectivement 435, à travers la plaque de mousse, respectivement droite 436 et gauche 437, pour maintenir en prise la partie amovible 400 du dispositif de prise vue 200 et être escamoté lorsque celui-ci est contre la face extérieure de la paroi transparente 15.

Chaque plot inox magnétique 303, respectivement 304, coopère mécaniquement avec le logement vertical 434, respectivement 435.

Chaque plot inox magnétique 303, respectivement 304, coopère magnétiquement avec deux ensembles d'aimants, respectivement 444 et 445, logés dans des logements de chacune des bases, respectivement 416 et 417, vers l'avant de la partie amovible 400, en regard des logements 434 et 435.

La face avant comporte un support sur lequel une plaque 453 de logo est collée.

La face avant comporte en outre quatre ouvertures de ventilation. Des filtres 454 sont disposés à l'intérieur du capot 448 et contre les ouvertures de ventilation.

La face avant est écornée de sorte que deux emplacements pour coins rapportés sont prévus. Deux types de coins pouvant être rapportés sur les emplacements sont prévus.

Dans l'exemple représenté, un coin 455 avec passe fil est prévu pour être placé sur le côté droit. Un coin 456 sans passe fil est prévu pour être placé sur le côté gauche. Chacun des coins est fixé sur l'ensemble mécanique 401 au moyen d'une vis inox 457.

Le capot est en outre fixé sur la face inférieure de la base gauche 416, respectivement de la base droite 417, au moyen de vis inviolables 458.

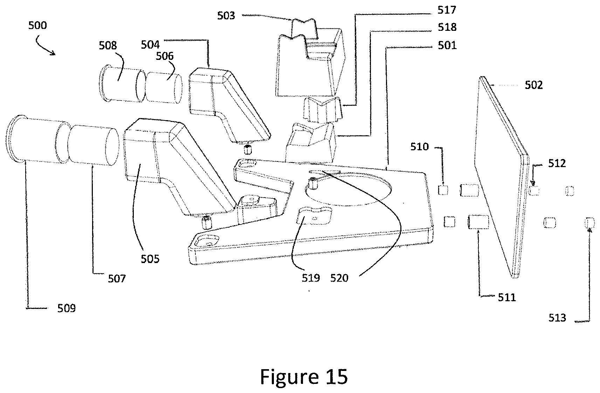

Les figures 14 et 15 illustrent le support 500 de positionnement d'objets.

Le support 500 de positionnement d'objet comporte une base 501 plane de support, un écran 502 dressé en arrière de la base 501 destiné à former un fond blanc, un adaptateur 503 pour petits objets, monté sur la base 501 et deux pattes, respectivement droite 504 et gauche 505, s'étendant verticalement à l'avant de la base 501 et destinées à venir en appui contre la paroi interne de la baie 15.

Les pattes gauche 504 et droite 505 portent des moyens magnétiques, tels que des aimants, respectivement 506 et 507, complémentaires des deux ensembles d'aimants, respectivement 444 et 445.

Plus précisément, ces moyens magnétiques (pastille aimantée ou métallique, suivant celle utilisée pour les moyens magnétiques du dispositif de prise de vue) sont logés à l'intérieur de deux ergots 508 et 509 présentant une paroi avant arrondie pouvant se plaquer contre la face interne de la baie vitrée, au niveau des moyens magnétiques 444 et 445 du dispositif de prise de vue. L'arrondi de la paroi permet un contact ponctuel, ce qui autorise une distance minimale entre les moyens magnétiques et le plot en inox magnétique.

Ainsi, le positionnement du support 500 par rapport au dispositif de prise de vue 200 est précis.

L'écran 502 est monté de manière amovible par rapport à la base 501, au moyen d'aimants 510 logés dans des cages 511 de la base 501, complémentaires d'aimants 512 logés dans des bouchons 513 de l'écran 502.

L'adaptateur 503 pour petits objets présente une forme de pavé de forme sensiblement rectangulaire comportant deux chevalets 514, 515, définissant une section transversale en forme de M, afin de servir de support pour le corps d'une seringue, et de retenir latéralement par les pans inclinés, ce corps de seringue.

L'adaptateur 503 comporte en outre un logement d'accueil 516, disposé entre les deux chevalets, pour le cul d'un flacon.

L'adaptateur 503 est positionné, de manière amovible, sur la base 501 par l'intermédiaire de deux pattes, respectivement 517 et 518, s'étendant verticalement, lesquelles viennent se loger dans deux logements formés dans la base 501, respectivement 519 et 520.

Lorsque l'adaptateur 503 est retiré, les pattes 517 et 518 sont elles-mêmes des chevalets, définissant une section transversale en forme de M, afin de servir de support pour le corps d'une seringue et de retenir latéralement par les pans inclinés, ce corps de seringue.

Les pattes 517 et 518 sont utilisées de préférence en tant que support de grosses seringues.

En outre, l'adaptateur 503 comporte un élément de référence, tel qu'un codebarres, disposé, par exemple, sur une partie frontale, et visible par une caméra. La présence ou l'absence de l'élément de référence peut être détectée par une unité de traitement et sélectionne un mode de traitement particulier.

Bien sûr, l'invention n'est pas limitée aux exemples qui viennent d'être décrits et de nombreux aménagements peuvent être apportés à ces exemples sans sortir du cadre des revendications.

## Revendications

1. Dispositif (200) de prise de vue d'une pluralité d'objets tels qu'un flacon et une seringue, en vue de l'élaboration sécurisée de préparations médicamenteuses, comprenant :
- au moins une paire d'appareils (404, 405) de prise de vue disposés en regard l'un de l'autre,
- un élément réfléchissant (406, 407) pour chacun des appareils de prise de vue,
les deux éléments réfléchissants d'une même paire d'appareils, étant disposés entre lesdits appareils et chacun orienté de façon à réfléchir en direction de l'appareil correspondant, des images d'une zone d'élaboration d'une préparation médicamenteuse,
le dispositif de prise de vue comportant en outre :
- une partie fixe (300) formée par des moyens de positionnement (301, 302) destinés à être fixés à demeure sur une enceinte (15) de la zone d'élaboration de la préparation médicamenteuse,
- une partie amovible (400), destinée à être fixée de manière amovible, sur la partie fixe, ladite partie amovible comportant au moins une paire d'appareils de prise de vue et l'élément réfléchissant,
dispositif **caractérisé en ce que**
la partie amovible comporte une base (416, 417) destinée à être fixée de manière amovible sur la partie fixe et une demi-embase (402, 403) montée à rotation par rapport à la base, la demi-embase comportant au moins l'un des appareils de prise de vue,
chacune des bases comporte, du côté arrière de la base, un logement vertical (434, 435), de forme oblongue présentant un trou de centrage de forme tronconique en son centre, d'axe perpendiculaire à la forme oblongue, et
chacun des deux moyens de positionnement comporte un plot inox magnétique (303, 304), chaque plot inox magnétique coopérant mécaniquement avec le logement vertical.

2. Dispositif de prise de vue selon la revendication précédente, dans lequel les moyens de positionnement (301, 302) coopèrent avec des moyens magnétiques complémentaires (444, 445) disposés sur la partie amovible.

3. Dispositif selon la revendication précédente, comportant en outre des moyens de sélection (424, 427) de l'angle formé entre la base (416, 417) et la demi-embase (402, 403).

4. Dispositif de prise de vue selon l'une quelconque des revendications précédentes, dans lequel les deux zones couvertes par les deux appareils d'une même paire d'appareils (404, 405) sont spatialement décalées, et en ce que les appareils présentent des objectifs de focales différentes permettant de faire la mise au point sur des objets de tailles différentes se trouvant dans la zone d'élaboration de la préparation médicamenteuse.

5. Dispositif de prise de vue selon l'une quelconque des revendications précédentes, dans lequel la partie amovible (400) comporte un capot (448) qui est lui-même amovible par rapport à ladite partie amovible (400).

6. Dispositif de prise de vue selon l'une quelconque des revendications précédentes, dans lequel les deux éléments réfléchissants (406, 407) sont formés par des surfaces planes.

7. Système (100) d'imagerie sécurisé, comprenant un dispositif (200) de prise de vue selon l'une quelconque des revendications précédentes, un support (500) de positionnement d'objets, et une enceinte d'élaboration de préparations médicamenteuses, le support de positionnement d'objets étant disposé à l'intérieur de l'enceinte, et le dispositif de prise de vue à l'extérieur de celle-ci contre une baie transparente de l'enceinte, et à une position telle que des images du support de positionnement d'objets, parviennent aux appareils de prise de vue du dispositif.

8. Système selon la revendication précédente, dans lequel les moyens de fixation (506, 507) du support de positionnement d'objets sont des moyens magnétiques qui coopèrent à travers l'enceinte (15) de préparation médicamenteuse, avec les moyens de positionnement (301, 302) disposés sur la partie fixe (300) du dispositif de prise de vue (200).

9. Système selon la revendication précédente, lorsque le dispositif est réalisé selon la revendication 2, dans lequel les moyens de fixation (506, 507) du support de positionnement d'objets sont des moyens magnétiques qui coopèrent avec les moyens magnétiques complémentaires (444, 445) disposés sur la partie amovible (400) du dispositif de prise de vue (200).

10. Système selon l'une quelconque des revendications précédentes de système, dans lequel le support de positionnement d'objet comporte en outre une base plane (501) de support des objets et un écran (502) dressé en arrière de la base plane, l'écran étant amovible par rapport à la base plane, l'écran comportant des moyens magnétiques (512) complémentaires de moyens magnétiques (510) logés dans la base plane.

## Patentansprüche

1. Vorrichtung (200) für eine Bildaufnahme einer Vielzahl von Objekten, wie etwa einer Flasche und einer Spritze, im Hinblick auf die sichere Herstellung von Arzneimittelzubereitungen, umfassend:
- mindestens ein Paar von Bildaufnahmeeinrichtungen (404, 405), die einander gegenüberliegend angeordnet sind,
- ein reflektierendes Element (406, 407) für jede der Bildaufnahmeeinrichtungen,
wobei die zwei reflektierenden Elemente eines gleichen Paars von Einrichtungen zwischen den Einrichtungen angeordnet und jeweils ausgerichtet sind, um Bilder eines Bereichs für die Herstellung einer Arzneimittelzubereitung in Richtung der entsprechenden Einrichtung zu reflektieren,
wobei die Bildaufnahmevorrichtung ferner aufweist:
- einen festen Teil (300), der durch Positionierungsmittel (301, 302) ausgebildet ist, die dafür bestimmt sind, an einem Gehäuse (15) des Bereichs für die Herstellung der Arzneimittelzubereitung dauerhaft befestigt zu werden,
- einen abnehmbaren Teil (400), der dafür bestimmt ist, auf abnehmbare Weise an dem festen Teil befestigt zu werden, wobei der abnehmbare Teil mindestens ein Paar von Bildaufnahmeeinrichtungen und das reflektierende Element aufweist, wobei die Vorrichtung **dadurch gekennzeichnet ist, dass**
der abnehmbare Teil eine Basis (416, 417), die dafür bestimmt ist, auf abnehmbare Weise an dem festen Teil befestigt zu werden, und eine Halbbasisplatte (402, 403) aufweist, die relativ zu der Basis drehbar montiert ist, wobei die Halbbasisplatte mindestens eine der Bildaufnahmeeinrichtungen aufweist,
jede der Basen auf der Rückseite der Basis eine vertikale Aufnahme (434, 435) mit länglicher Form aufweist, die ein Zentrierloch mit kegelstumpfartiger Form in ihrem Zentrum mit einer Achse senkrecht zu der länglichen Form vorweist, und jedes der zwei Positionierungsmittel einen magnetischen Edelstahlstift (303, 304) aufweist, wobei jeder magnetische Edelstahlstift mit der vertikalen Aufnahme mechanisch zusammenwirkt.

2. Bildaufnahmevorrichtung nach dem vorstehenden Anspruch, wobei die Positionierungsmittel (301, 302) mit komplementären magnetischen Mitteln (444, 445) zusammenwirken, die an dem abnehmbaren Teil angeordnet sind.

3. Vorrichtung nach dem vorstehenden Anspruch, die ferner Mittel (424, 427) zum Auswählen des Winkels aufweist, der zwischen der Basis (416, 417) und der Halbbasisplatte (402, 403) ausgebildet ist.

4. Bildaufnahmevorrichtung nach einem der vorstehenden Ansprüche, wobei die zwei Bereiche, die durch die zwei Einrichtungen eines gleichen Paars von Einrichtungen (404, 405) abgedeckt werden, räumlich versetzt sind, und dass die Einrichtungen Objektive unterschiedlicher Brennweiten vorweisen, die die Fokussierung auf Objekte mit unterschiedlichen Größen ermöglichen, die sich in dem Bereich für die Herstellung der Arzneimittelzubereitung befinden.

5. Bildaufnahmevorrichtung nach einem der vorstehenden Ansprüche, wobei der abnehmbare Teil (400) eine Haube (448) aufweist, die wiederum relativ zu dem abnehmbaren Teil (400) abnehmbar ist.

6. Bildaufnahmevorrichtung nach einem der vorstehenden Ansprüche, wobei die zwei reflektierenden Elemente (406, 407) durch ebene Oberflächen ausgebildet sind.

7. Sicheres Bildgebungssystem (100), umfassend eine Bildaufnahmevorrichtung (200) nach einem der vorstehenden Ansprüche, eine Objektpositionierungshalterung (500) und ein Gehäuse für die Herstellung von Arzneimittelzubereitungen, wobei die Objektpositionierungshalterung innerhalb des Gehäuses und die Bildaufnahmevorrichtung außerhalb dessen gegen eine transparente Öffnung des Gehäuses und in einer solchen Position angeordnet ist, dass Bilder der Objektpositionierungshalterung zu den Bildaufnahmeeinrichtungen der Vorrichtung gelangen.

8. System nach dem vorstehenden Anspruch, wobei die Befestigungsmittel (506, 507) der Objektpositionierungshalterung magnetische Mittel sind, die durch das Gehäuse (15) für die Arzneimittelzubereitung hindurch mit den Positionierungsmitteln (301, 302) zusammenwirken, die an dem festen Teil (300) der Bildaufnahmevorrichtung (200) angeordnet sind.

9. System nach dem vorstehenden Anspruch, wenn die Vorrichtung nach Anspruch 2 ausgeführt ist, wobei die Befestigungsmittel (506, 507) der Objektpositionierungshalterung magnetische Mittel sind, die mit den komplementären magnetischen Mitteln (444, 445) zusammenwirken, die an dem abnehmbaren Teil (400) der Bildaufnahmevorrichtung (200) angeordnet sind.

10. System nach einem der vorstehenden Systemansprüche, wobei die Objektpositionierungshalterung ferner eine ebene Basis (501) zum Halten der Objekte und einen Bildschirm (502) aufweist, der hinter der ebenen Basis aufgerichtet ist, wobei der Bildschirm relativ zu der ebenen Basis abnehmbar ist, wobei der Bildschirm magnetische Mittel (512) aufweist, die komplementär zu den magnetischen Mitteln (510) sind, die in der ebenen Basis aufgenommen sind.

## Claims

1. Device (200) for capturing an image of a plurality of objects such as a bottle and a syringe, for the secure production of medicinal preparations, including:
- at least one pair of image capturing apparatuses (404, 405) arranged opposite one another,
- a reflecting element (406, 407) for each of the image capturing apparatuses,
the two reflecting elements of a given pair of apparatuses being arranged between said apparatuses and each being oriented so as to reflect, toward the corresponding apparatus, images of an area for producing a medicinal preparation,
the image capturing device further comprising:
- a stationary part (300) formed by positioning means (301, 302) that are intended to be secured permanently against an enclosure (15) of the area for producing the medicinal preparation,
- a removable part (400) that is intended to be removably secured to the stationary part, said removable part comprising at least one pair of image capturing apparatuses and the reflecting element, the device being **characterized in that**
the removable part comprises a base (416, 417) that is intended to be removably secured to the stationary part and a half-baseplate (402, 403) that is mounted so as to rotate relative to the base, the half-baseplate comprising at least one of the image capturing apparatuses,
each of the bases comprises, on the rear side of the base, a vertical recess (434, 435) having an oblong shape and having a centering hole having a frustoconical shape at its center and having an axis that is perpendicular to the oblong shape, and
each of the two positioning means comprises a magnetic stainless steel stud (303, 304), each magnetic stainless steel stud interacting mechanically with the vertical recess.

2. Image capturing device according to the preceding claim, wherein the positioning means (301, 302) interact with complementary magnetic means (444, 445) arranged on the removable part.

3. Device according to the preceding claim, further comprising means (424, 427) for selecting the angle formed between the base (416, 417) and the half-baseplate (402, 403).

4. Image capturing device according to any of the preceding claims, wherein the two areas covered by the two apparatuses of a given pair of apparatuses (404, 405) are spatially offset, and wherein the apparatuses have lenses of different focal lengths, making it possible to focus on objects of different sizes located in the area for producing the medicinal preparation.

5. Image capturing device according to any of the preceding claims, wherein the removable part (400) comprises a cover (448) which is itself removable with respect to said removable part (400).

6. Image capturing device according to any of the preceding claims, wherein the two reflecting elements (406, 407) are formed by planar surfaces.

7. Secure imaging system (100), including an image capturing device (200) according to any of the preceding claims, an object-positioning support (500) and an enclosure for producing medicinal preparations, the object-positioning support being arranged inside the enclosure, and the image capturing device being positioned outside said enclosure against a transparent window of the enclosure, and in a position such that images of the object-positioning support reach the image capturing apparatuses of the device.

8. System according to the preceding claim, wherein the means (506, 507) for securing the object-positioning support are magnetic means which interact, through the medicinal preparation enclosure (15), with the positioning means (301, 302) arranged on the stationary part (300) of the image capturing device (200).

9. System according to the preceding claim, when the device is produced according to claim 2, wherein the means (506, 507) for securing the object-positioning support are magnetic means which interact with the complementary magnetic means (444, 445) arranged on the removable part (400) of the image capturing device (200).

10. System according to any of the preceding system claims, wherein the object-positioning support further comprises a planar base (501) for supporting the objects and a screen (502) erected behind the planar base, the screen being removable with respect to the planar base, the screen comprising magnetic means (512) that are complementary to magnetic means (510) accommodated in the planar base.
